(19) Europäisches Patentamt
European Patent Office
Office européen des brevets

(11) Veröffentlichungsnummer: **0 096 815**
**B1**

(12) # EUROPÄISCHE PATENTSCHRIFT

(45) Veröffentlichungstag der Patentschrift: **08.05.85**

(51) Int. Cl.⁴: **C 07 C 103/44**

(21) Anmeldenummer: **83105535.5**

(22) Anmeldetag: **06.06.83**

(54) **Verfahren zur Herstellung von kernsubstituierten N-Alkylanilinen.**

(30) Priorität: **11.06.82 DE 3222013**

(43) Veröffentlichungstag der Anmeldung: **28.12.83 Patentblatt 83/52**

(45) Bekanntmachung des Hinweises auf die Patenterteilung: **08.05.85 Patentblatt 85/19**

(84) Benannte Vertragsstaaten: **CH DE FR GB IT LI**

(56) Entgegenhaltungen:
**CH - A - 597 160**
**FR - A - 2 409 288**
**FR - A - 2 432 537**

(73) Patentinhaber: **HOECHST AKTIENGESELLSCHAFT, Postfach 80 03 20, D-6230 Frankfurt am Main 80 (DE)**

(72) Erfinder: **Sommer, Karl, Dr., Thewaltstrasse 6, D-6240 Königstein/Taunus (DE)**
Erfinder: **Schickfluss, Rudolf, Dr., Luisenstrasse 37, D-6233 Kelkheim (Taunus) (DE)**

**EP 0 096 815 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Gegenstand der vorliegenden Erfindung ist ein Verfahren zur Herstellung von kernsubstituierten N-Alkyl-anilinen durch reduktive Alkylierung von kernsubstituierten Amino- oder Nitrobenzolen.

Über die Herstellung von sekundären Aminen der Anilinreihe durch reduktive Alkylierung gibt es eine Fülle von Veröffentlichungen (vgl. hierzu Houben-Weyl, Band XI/1, S. 618 ff., 4. Auflage, Stuttgart, 1957). Danach werden aromatische, primäre Aminoverbindungen mit geeigneten Carbonylverbindungen, die auch als Reaktionsmedium dienen können, in Gegenwart von Wasserstoff alkyliert. Die Reaktionsbedingungen zeigen eine große Variationsbreite.

Bei der Durchsicht der umfangreichen Patentliteratur fällt auf, daß es sich bei vielen Veröffentlichungen um die N-Alkylierung des Grundkörpers Anilin oder von einfach kernsubstituierten Anilinen handelt, [DE-PS 2 153 792, US-PS 3 509 213, DE-OS 2 941 070]. Von dem gemäß dem Verfahren der DE-OS 2 745 552 erhältlichen aromatischen Aminen unterscheiden sich die erfindungsgemäß erhältlichen Verbindungen durch den Gehalt eines Alkoxy-alkoxy-Restes in o-Stellung zur N-alkylierten Aminogruppe. Ferner werden bei dem Verfahren der letztgenannten Literaturstelle als Carbonylverbindungen ausschließlich Aldehyde eingesetzt.

Es wurde nun gefunden, daß man kernsubstituierte N-Alkyl-aniline der allgemeinen Formel (1)

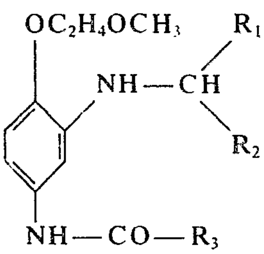 (1)

in welcher $R_1$, $R_2$ und $R_3$ die Methyl- oder Äthylgruppe bedeuten, in guten Ausbeuten herstellen kann, indem man eine Verbindung der allgemeinen Formel (2)

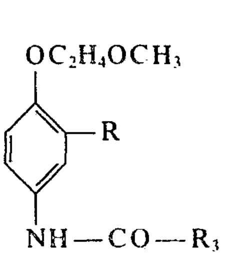 (2)

in welcher R die Amino- oder Nitrogruppe und $R_3$ die Methyl- oder Äthylgruppe bedeuten, mit einer aliphatischen Carbonylverbindung der Formel (3)

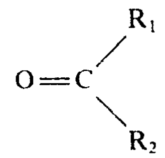 (3)

in welcher $R_1$ und $R_2$ die vorstehend genannten Bedeutungen haben, in Gegenwart eines sauren Reaktionsbeschleunigers und eines Nickelkatalysators bei Temperaturen von 100–180°C bei einem Wasserstoffdruck von 20–150 bar, vorzugsweise von 140–160°C und einem Wasserstoffdruck von 50–100 bar, reduktiv alkyliert.

Die Reaktionszeit im Autoklav beträgt zwischen 3 und 7 Stunden.

Als saure Reaktionsbeschleuniger können beispielsweise Essigsäure, Propionsäure, Benzolsulfonsäure, p-, m- oder o-Toluolsulfonsäure oder Gemische aus den genannten stellungsisomeren Toluolsulfonsäuren oder Xylolsulfonsäuren verwendet werden.

Geeignete Nickelkatalysatoren sind vorzugsweise teilstabilisierte Nickelkatalysatoren auf Träger (Kohle oder Aluminiumoxid) mit etwa 50%igem Nickelanteil.

Nach beendeter Reaktion wird der Katalysator abgetrennt und das Reaktionsgemisch der Vakuumdestillation unterworfen. Die zurückbleibende, hinsichtlich des jeweiligen gewünschten Endprodukts etwa 75%ige Schmelze kann dann als solche sofort weiterverarbeitet werden. Alternativ kann durch Zusatz von Wasser oder wäßriger Salzlösung zur Schmelze das darin enthaltene Endprodukt der Formel (1) abgeschieden werden.

Es ist als überraschend zu erachten, daß trotz des relativ großen Substituenten in o-Stellung zur Amino- bzw. Nitrogruppe der Verbindung der allgemeinen Formel (2) die reduktive Alkylierung glatt und mit guten Ausbeuten abläuft und unter den gewählten Bedingungen sowohl N-Dialkylierung als auch Kernhydrierung vermieden werden. Die für die Herstellung von Farbstoffen wertvollen Verbindungen der allgemeinen Formel (1) werden in einer Qualität erhalten, die eine sofortige Weiterverarbeitung zum Farbstoff ermöglicht.

Die nachstehenden Beispiele illustrieren das erfindungsgemäße Verfahren ohne es darauf zu beschränken.

Beispiel 1

238 Gewichtsteile 2-Amino-4-propionylamino-1-β-methoxyäthoxy-benzol werden in 800 Volumenteilen Aceton unter Zugabe von 30 Volumenteilen Propionsäure mit 10 Gewichtsteilen Nickelkatalysator in einem Autoklav 6 Stunden bei 140°C und ca. 100 bar Wasserstoff reduktiv alkyliert. Nach dem Abtrennen des Katalysators wird das Lösungsmittelgemisch unter Vakuum weitgehend abgezogen und das als ca. 75%ige

Schmelze vorliegende Reaktionsprodukt als solches weiter verarbeitet oder durch Eintropfen von Wasser oder Kochsalzlösung abgeschieden. Auf diese Weise erhält man die Verbindung der allgemeinen Formel (1) mit $R_1 = R_2$ = Methyl und mit $R_3$ = Äthyl in einer Ausbeute von 90% der Theorie.

Das Schmelzintervall liegt bei 72–73°C.

Beispiel 2

224 Gewichtsteile 2-Amino-4-acetamino-1-β-methoxyäthoxybenzol werden in 800 Volumenteilen Methyläthylketon mit 60 Volumenteilen Essigsäure und 20 Gewichtsteilen Nickelkatalysator in einem Autoklav 6 Stunden bei 150°C und ca. 100 bar Wasserstoff reduktiv alkyliert. Die Aufbereitung erfolgt wie in Beispiel 1 beschrieben. Man erhält die Verbindung der allgemeinen Formel (1) mit $R_1$ und $R_3$ = Methyl und $R_2$ = Äthyl in einer Ausbeute von 87% der Theorie.

Der Schmelzpunkt beträgt 89°C.

Beispiel 3

254 Gewichtsteile 2-Nitro-4-acetamino-1-β-methoxyäthoxybenzol werden in 800 Volumenteilen Aceton unter Zugabe von 3 Gewichtsteilen p-Toluolsulfonsäure mit 15 Gewichtsteilen Nickelkatalysator in einem Autoklav 5 Stunden bei 160°C und ca. 50 bar Wasserstoff reduktiv alkyliert. Die Aufbereitung erfolgt wie in Beispiel 1 beschrieben. Man erhält die Verbindung der allgemeinen Formel (1) mit $R_1$, $R_2$ und $R_3$ = Methyl in einer Ausbeute von 87% der Theorie.

Das Schmelzintervall liegt bei 116–117°C.

Beispiel 4

238 Gewichtsteile 2-Amino-4-propionylamino-1-β-methoxyäthoxy-benzol werden in 800 Volumenteilen Diäthylketon mit 10 Volumenteilen Essigsäure und 20 Gewichtsteilen Nickelkatalysator in einem Autoklav 6 Stunden bei 150°C und ca. 80 bar Wasserstoff reduktiv alkyliert. Die Aufbereitung erfolgt wie in Beispiel 1 beschrieben. Man erhält die Verbindung der allgemeinen Formel (1) mit $R_1$, $R_2$ und $R_3$ = Äthyl in einer Ausbeute von 91% der Theorie.

Das Schmelzintervall liegt bei 65–66°C.

**Patentansprüche**

1. Verfahren zur Herstellung von N-Alkyl-anilinen der allgemeinen Formel (1)

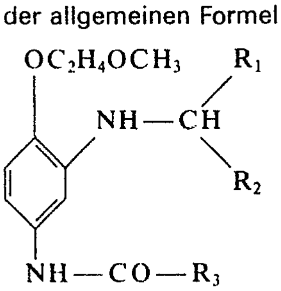

(1)

in welcher $R_1$, $R_2$ und $R_3$ die Methyl- oder Äthylgruppe bedeuten, dadurch gekennzeichnet, daß man eine Verbindung der allgemeinen Formel (2)

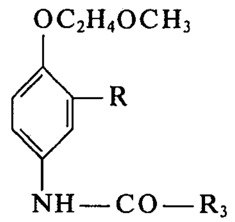

(2)

in welcher R die Amino- oder Nitrogruppe und $R_3$ die Methyl- oder Äthylgruppe bedeuten, mit einer aliphatischen Carbonylverbindung der Formel (3)

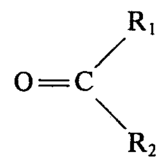

(3)

in welcher $R_1$ und $R_2$ die vorstehend genannten Bedeutungen haben, in Gegenwart eines sauren Reaktionsbeschleunigers und eines Nickelkatalysators bei Temperaturen von 100–180°C bei einem Wasserstoffdruck von 20–150 bar reduktiv alkyliert.

2. Verfahren nach Anspruch 1, dadurch gekennzeichnet, daß man das Verfahren in Gegenwart von Essigsäure, Propionsäure, Benzolsulfonsäure, p-, m- oder o-Toluolsulfonsäure oder Mischungen aus diesen Toluolsulfonsäuren oder einer Xylolsulfonsäure durchführt.

**Claims**

1. A process for the preparation of N-alkyl-anilines of the general formula (1)

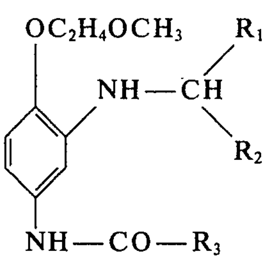

(1)

in which $R_1$, $R_2$ and $R_3$ denote the methyl or ethyl group, which comprises subjecting a compound of the general formula (2)

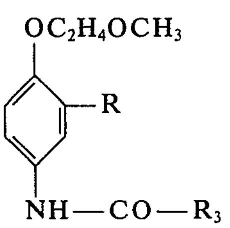

(2)

in which R denotes the amino or nitro group and $R_3$ denotes the methyl or ethyl group, to reductive alkylation with an aliphatic carbonyl compound of the formula (3)

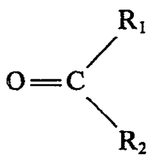 (3)

in which $R_1$ and $R_2$ have the abovementioned meanings, in the presence of an acid reaction accelerator and a nickel catalyst at temperatures of 100–180°C under a hydrogen pressure of 20–150 bar.

2. A process as claimed in claim 1, wherein the process is carried out in the presence of acetic acid, propionic acid, benzenesulfonic acid, p-, m- or o-toluenesulfonic acid, a mixture of these toluenesulfonic acids or a xylene-sulfonic acid.

**Revendications**

1. Procédé de préparation de N-alkyl-anilines répondant à la formule générale (1):

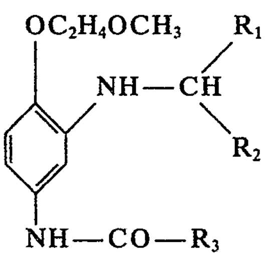 (1)

dans laquelle $R_1$, $R_2$ et $R_3$ représentent chacun un radical méthyle ou éthyle, procédé caractérisé en ce qu'on soumet à une alkylation réductrice un composé répondant à la formule générale (2)

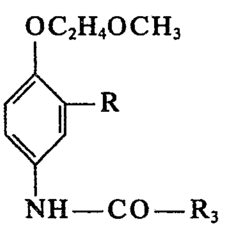 (2)

dans laquelle R représente un radical amino ou nitro et $R_3$ représente un radical méthyle ou éthyle, avec un composé carbonylique aliphatique répondant à la formule (3):

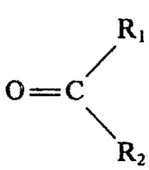 (3)

dans laquelle $R_1$ et $R_2$ ont les significations précédemment données, en présence d'un accélérateur de réaction acide et d'un catalyseur au nickel, à des températures de 100 à 180°C et sous une pression d'hydrogène de 20 à 150 bar.

2. Procédé selon la revendication 1, caractérisé en ce qu'on effectue le procédé en présence d'acide acétique, d'acide propionique, d'acide benzène-sulfonique, d'acide p-, m- ou o-toluène-sulfonique, d'un mélange de ces acides toluène-sulfoniques ou d'un acide xylène-sulfonique.